(19) Europäisches Patentamt / European Patent Office / Office européen des brevets

(11) **EP 3 082 459 B1**

(12) **FASCICULE DE BREVET EUROPEEN**

(45) Date de publication et mention de la délivrance du brevet:
**30.01.2019 Bulletin 2019/05**

(21) Numéro de dépôt: **14821241.8**

(22) Date de dépôt: **27.11.2014**

(51) Int Cl.:
*A23K 50/42* *(2016.01)*      *A23K 10/30* *(2016.01)*
*A21D 13/80* *(2017.01)*      *A23L 27/60* *(2016.01)*

(86) Numéro de dépôt international:
**PCT/FR2014/053052**

(87) Numéro de publication internationale:
**WO 2015/079169 (04.06.2015 Gazette 2015/22)**

(54) **GRANULES DE FARINE DE BIOMASSE DE MICROALGUES RICHES EN PROTÉINES ET LEUR PROCÉDÉ DE PRÉPARATION**

GRANULAT AUS PROTEINREICHEM MIKROALGENBIOMASSEMEHL UND VERFAHREN ZUR HERSTELLUNG DAVON

GRANULES OF PROTEIN-RICH MICROALGAL BIOMASS FLOUR AND METHOD FOR PREPARING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.11.2013  FR 1361877**
**27.06.2014  FR 1456034**

(43) Date de publication de la demande:
**26.10.2016  Bulletin 2016/43**

(73) Titulaire: **Corbion Biotech, Inc.**
**South San Francisco, CA 94080 (US)**

(72) Inventeurs:
• **PASSE, Damien**
  **F-59500 Douai (FR)**
• **DELANNOY, François**
  **F-62157 Allouagne (FR)**

(74) Mandataire: **Cabinet Becker et Associés**
**25, rue Louis le Grand**
**75002 Paris (FR)**

(56) Documents cités:
**WO-A1-2014/062882    DE-A1-102006 056 454**

• **MÓNICA FRADIQUE ET AL: "Incorporation of Chlorella vulgaris and Spirulina maxima biomass in pasta products. Part 1: Preparation and evaluation", JOURNAL OF THE SCIENCE OF FOOD AND AGRICULTURE, vol. 90, no. 10, 15 août 2010 (2010-08-15) , pages 1656-1664, XP055027243, ISSN: 0022-5142, DOI: 10.1002/jsfa.3999**
• **NALIN SAMARASINGHE ET AL: "Algal cell rupture using high pressure homogenization as a prelude to oil extraction", RENEWABLE ENERGY, PERGAMON PRESS, OXFORD, GB, vol. 48, 20 avril 2012 (2012-04-20), pages 300-308, XP028428119, ISSN: 0960-1481, DOI: 10.1016/J.RENENE.2012.04.039 [extrait le 2012-05-11]**
• **SPIDEN ERIN M ET AL: "Quantitative evaluation of the ease of rupture of industrially promising microalgae by high pressure homogenization", BIORESOURCE TECHNOLOGY, ELSEVIER BV, GB, vol. 140, 28 avril 2013 (2013-04-28), pages 165-171, XP028565382, ISSN: 0960-8524, DOI: 10.1016/J.BIORTECH.2013.04.074**
• **BECKER ET AL: "Micro-algae as a source of protein", BIOTECHNOLOGY ADVANCES, ELSEVIER PUBLISHING, BARKING, GB, vol. 25, no. 2, 26 janvier 2007 (2007-01-26), pages 207-210, XP005862318, ISSN: 0734-9750, DOI: 10.1016/J.BIOTECHADV.2006.11.002**

**Description**

La présente invention est relative à des granules de farine de biomasse de microalgues riches en protéines, biomasse de microalgues intactes (non broyées).

Plus particulièrement, la présente invention est relative à des granules de farine de biomasse de microalgues présentant une distribution granulométrique, des propriétés de compressibilité et de densité aérée tout à fait remarquables.

La présente invention porte sur des granules de farine de biomasse de microalgues présentant des propriétés d'écoulement et de mouillabilité tout à fait satisfaisants.

La présente invention concerne également le procédé de préparation de ces granules de farine de biomasse de microalgues riches en protéines.

La présente invention porte enfin sur l'utilisation des granules de farine de biomasse de microalgues en alimentations humaine et animale (animaux de compagnie, aquaculture...), ou pour des applications dans l'industrie pharmaceutique et cosmétique.

Il existe plusieurs espèces d'algues qui peuvent être utilisées en Alimentaire, la plupart étant des « macroalgues » comme le varech, la laitue de mer (*Ulva lactuca*) et les algues rouges alimentaires de type *Porphyra* (cultivées au Japon) ou « dulse » (algue rouge *Palmaria palmata*).

Mais à côté de ces macroalgues, on trouve également de nombreuses sources d'algues que sont les « microalgues », notamment les algues microscopiques unicellulaires, photosynthétiques ou non, d'origine marine ou non, cultivées pour leurs applications en Bio-carburant ou Alimentaire.

Par exemple, la spiruline (*Arthrospira platensis*) est cultivée dans des lagunes ouvertes (en phototrophie) pour une utilisation comme complément alimentaire ou incorporée en petites quantités dans des confiseries ou des boissons (généralement moins de 0,5 % p/p).

D'autres microalgues, y compris certaines espèces de *Chlorella,* sont également très populaires dans les pays asiatiques comme compléments alimentaires.

La présente invention est ainsi relative à la biomasse de microalgues propre (ou adaptée) à la consommation humaine qui est riche en nutriments, notamment en protéines.

L'invention se rapporte à une farine de biomasse de microalgues riches en protéines qui peut être incorporée dans les produits alimentaires dans lesquels la teneur en protéines de la farine de microalgues peut se substituer en tout ou partie des protéines présentes dans les produits alimentaires conventionnels.

La farine de biomasse de microalgues fournit également d'autres bénéfices, comme des micronutriments, des fibres alimentaires (glucides solubles et insolubles), des triglycérides, des phospholipides, des glycoprotéines, des phytostérols, tocophérols, tocotriénols, et du sélénium.

Au sens de l'invention, les microalgues considérées sont les espèces qui produisent des protéines à haute richesse.

La biomasse de microalgues comprend au moins 50 % en poids sec de protéines, de préférence entre 50 et 70 % en poids sec de protéines.

Les microalgues préférées de l'invention peuvent, quant à elles, croître en conditions hétérotrophiques (sur sucres comme source carbonée et en l'absence de lumière).

Les microalgues sont du genre *Chlorella* riches en protéines.

La Chlorelle est une microalgue unicellulaire verte, appartenant à l'embranchement des Chlorophytes.

De manière préférée, les microalgues utilisées selon l'invention sont de type *Chlorella sorokiniana* ou *Chlorella protothecoides.*

Les microalgues sont cultivées en milieu liquide pour produire la biomasse en tant que telle.

Selon l'invention, les microalgues sont cultivées dans un milieu contenant une source de carbone et une source d'azote en absence de lumière (conditions hétérotrophiques).

Les milieux de croissance solides et liquides sont généralement disponibles dans la littérature, et les recommandations pour la préparation des milieux particuliers qui conviennent à une grande variété de souches de microorganismes peuvent être aisément trouvées, par exemple, en ligne à http:// www.utex.org/, un site maintenu par l'Université du Texas à Austin pour sa collection de culture d'algues (UTEX).

La production de biomasse est réalisée en fermenteurs (ou bioréacteurs).

Les exemples spécifiques de bioréacteurs, les conditions de culture, et la croissance hétérotrophe et les méthodes de propagation peuvent être combinés de toute manière appropriée pour améliorer l'efficacité de la croissance microbienne et la production de protéines.

Pour préparer la biomasse pour une utilisation en alimentaire, la biomasse obtenue en fin de fermentation est concentrée ou récoltée du milieu de fermentation.

Au moment de la récolte de la biomasse de microalgues du milieu de fermentation, la biomasse comprend des cellules intactes pour l'essentiel en suspension dans un milieu de culture aqueux.

Pour concentrer la biomasse, on procède alors à une étape de séparation solide-liquide par filtration ou par centrifugation, prise seule ou en combinaison, par tout moyen connu par ailleurs de l'homme du métier.

Après concentration, la biomasse de microalgues peut être traitée afin de produire des gâteaux emballés sous vide, des paillettes d'algues, des homogénats d'algues, de la poudre d'algues, de la farine d'algues.

Conformément à l'invention, on procède au séchage de la biomasse de microalgues pour faciliter le traitement ultérieur ou pour une utilisation de la biomasse dans ses différentes applications.

Différentes textures et saveurs peuvent être conférées à des produits alimentaires, selon que la biomasse algale est séchée, et si oui, en fonction de la méthode de séchage mise en oeuvre.

Par exemple, le brevet US 6,607,900 décrit le séchage de la biomasse de microalgues en utilisant un séchoir à tambour sans centrifugation préalable, pour préparer des flocons (« flakes ») de microalgues.

De la poudre de microalgues peut être préparée à partir de la biomasse de microalgues concentré à l'aide d'un sécheur pneumatique ou par atomisation, comme décrit dans le brevet US 6,372,460. Le document DE102006056454 décrit un granulé comprenant trois types de microalgues, obtenu par atomisation.

Dans un atomiseur, une suspension liquide est alors pulvérisée sous la forme d'une dispersion de fines gouttelettes dans un courant d'air chauffé. Le matériel entraîné est rapidement séché et forme une poudre sèche.

Dans certains cas, un séchoir à combustion pulsé peut également être utilisé pour obtenir une texture poudreuse dans le matériau final séché.

Dans d'autres cas encore, une combinaison d'un séchage par atomisation suivi de l'utilisation d'un séchoir à lit fluidisé est mise en oeuvre pour atteindre les conditions optimales d'obtention d'une biomasse microbienne séchée (voir, par exemple, le brevet US 6,255,505).

Dans le domaine technique auquel s'adresse l'invention, on recherche à préparer une farine de biomasse d'algues à partir de la biomasse de microalgues concentrée puis atomisée ou flash-séchée.

Après séchage, la teneur en eau ou l'humidité de la poudre est généralement inférieure à 10 % en poids.

Cependant, dans les procédés classiques de récupération de la biomasse de microalgues riches en protéines, on peut déplorer l'obtention d'une poudre sèche de faible compressibilité et de faible densité aérée.

**Il existe donc encore un besoin non satisfait** pour de nouvelles formes de farine de biomasse de microalgues riches en protéines afin de permettre leur incorporation aisée, à grande échelle, dans des produits alimentaires qui doivent rester savoureux et nutritifs.

La société Demanderesse a donc trouvé que ce besoin pouvait être satisfait en proposant des granules de farine de biomasse de microalgues présentant une distribution granulométrique, des propriétés de compressibilité et de densité aérée tout à fait remarquables.

Les granules de farine de biomasse de microalgues conformes à l'invention sont ainsi caractérisés en ce qu'ils présentent :

- une distribution granulométrique, mesurée sur un granulomètre laser LS de marque COULTER®, présentant un Dmode compris entre 60 et 300 $\mu$m et un D4,3 entre 70 et 420 $\mu$m,
- une densité aérée, mesurée sur Powder Characteristics Tester HOSOKAWA, comprise entre 0,60 et 0,70 g/ml,
- une compressibilité, mesurée sur Powder Characteristics Tester HOSOKAWA, comprise entre 15 et 25 %, de préférence entre 18 et 21 %.

Les granules de farine de biomasse de microalgues selon l'invention sont d'abord caractérisés par leur distribution granulométrique.

Cette mesure est réalisée sur un granulomètre laser LS de marque COULTER®, muni de son module de dispersion petit volume ou SVM (125 ml) en suivant les spécifications du constructeur (dans les *« Small Volume Module Operating instructions »*).

Les répartitions granulométriques sont illustrées par les valeurs de Dmode (diamètre de la population principale) et celles de D4,3 (diamètre moyen arithmétique).

Les granules de farine de biomasse de microalgues conformes à l'invention ont alors une distribution granulométrique monomodale, caractérisée par un Dmode compris entre 60 et 300 $\mu$m et un D4,3 entre 70 et 420 $\mu$m.

Plus particulièrement, les granules de farine de biomasse de microalgues selon l'invention peuvent être classées entre deux familles, en fonction de leur origine microalgale :

- la première famille de granules de farine de biomasse de *Chlorella sorokiniana,* présente un Dmode compris entre 70 et 130 $\mu$m et un D4,3 entre 75 et 140 $\mu$m ;
- la seconde famille de granules de farine de biomasse de *Chlorella protothecoides,* présente un Dmode compris entre 200 et 280 $\mu$m et un D4,3 entre 300 et 420 $\mu$m.

Les granules de farine de biomasse de microalgues conformes à l'invention présentent également une densité aérée, mesurée sur Powder Characteristics Tester HOSOKAWA, comprise entre 0,60 et 0,70 g/ml et une compressibilité, mesurée sur Powder Characteristics Tester HOSOKAWA, comprise entre 15 et 25 %, de préférence entre 18 et 21 %.

Les valeurs de densité tassée et aérée, et de compressibilité des granules de farine de biomasse de microalgues selon l'invention sont déterminées en utilisant l'appareil Powder Characteristics Tester type PTE commercialisé par la société HOSOKAWA, en suivant les spécifications du constructeur.

Cet appareil permet de mesurer, dans des conditions standardisées et reproductibles, l'aptitude à l'écoulement d'une poudre en mesurant notamment la densité aérée vrac et la densité tassée vrac et ensuite de calculer, à partir de ces données, les valeurs de compressibilité par la formule suivante :

$$\text{Compressibilité (\%)} = \frac{(\text{densité tassée - densité aérée})}{\text{densité tassée}} \times 100$$

Les mesures de densité tassée et de densité aérée sont réalisées sur l'appareil Powder Characteristics Tester de type PTE, comme mentionné ci-avant, selon la méthode préconisée dans le mode d'emploi dudit POWDER TESTER (réglage par défaut sur 180 secousses pour la mesure de la densité tassée).

Cette valeur de densité aérée est d'autant plus remarquable que les granules de farine de biomasse de microalgues conformes à l'invention présentent une densité aérée plus élevée que la farine de microalgues séchées par voie classique.

En effet, il est admis que la densité d'un produit sera d'autant plus faible qu'il sera granulé par atomisation.

Or, quoique granulés, les produits conformes à l'invention présentent une densité aérée plus élevée qu'attendue : à titre de comparaison, comme il sera exemplifié ci-après, les densités aérées des poudres de farine de microalgues atomisée par voie classique présentent une densité aérée plus faible, comprise entre 0,35 et 0,50 g/ml.

Les granules de farine de biomasse de microalgues selon l'invention présentent également des propriétés d'écoulement tout à fait satisfaisantes, selon un test A.

Le test A consiste à mesurer le degré de cohésion des granules de farine de microalgues selon l'invention.

Ce test de cohésion est inspiré du test de cohésion également décrit dans les « Operating Instructions » du Powder Characteristics Tester type PTE commercialisé par la société HOSOKAWA.

Le test A consiste tout d'abord à tamiser les granules de farine de biomasse de microalgues selon l'invention sur un tamis d'ouverture de maille de 800 $\mu$m.

Les granules présentant une taille inférieure à 800 $\mu$m sont ensuite récupérés, introduits dans un récipient fermé et subissent un mélange par mouvement épicycloïdal à l'aide d'un mélangeur de laboratoire de marque TURBULA type T2C.

Par ce mélange, selon leurs propres caractéristiques, les granules de farine de biomasse de microalgues conformes à l'invention exprimeront leurs propensions à s'agglomérer ou à se repousser.

Les granules ainsi mélangés sont ensuite déposés sur une colonne de 3 tamis (2000 $\mu$m ; 1400 $\mu$m ; 800 $\mu$m) pour un nouveau tamisage.

Le tamisage terminé, le refus sur chaque tamis est quantifié et le résultat donne une illustration du caractère « cohésif » ou « collant » des granules de farine de biomasse de microalgues.

Ainsi, une poudre de granules à écoulement libre, donc peu cohésive, ne sera pratiquement pas arrêtée par les tamis de grande ouverture, mais le sera d'autant plus que les mailles desdits tamis seront resserrées.

Le protocole est le suivant :

- tamiser la quantité de produit nécessaire sur un tamis de 800 $\mu$m pour récupérer 50 g de produit de taille inférieure à 800 $\mu$m,
- introduire ces 50 g de granules de taille inférieure à 800 $\mu$m dans un bocal en verre de contenance de 1 litre (Ref. BVBL Verrerie Villeurbannaise-Villeurbanne France) et refermer le couvercle,
- disposer ce bocal dans le mélangeur TURBULA modèle T2C réglé sur la vitesse de 42 t/mn (Willy A. Bachofen Sarl-Sausheim-France) et mélanger pendant 5 minutes,
- préparer une colonne de 3 tamis (de marque Saulas -Diamètre 200 mm ; Paisy Cosdon - France) qui seront placés sur un tamiseur de marque Fritsch modèle Pulverisette type 00.502 ; détail du montage en partant du bas vers le haut : tamiseur, fond de tamis, tamis de 800 $\mu$m, tamis de 1400 $\mu$m, tamis de 2000 $\mu$m, couvercle du tamiseur.
- déposer la poudre issue du mélange sur le haut de la colonne (tamis de 2000 $\mu$m), fermer avec le couvercle du tamiseur et tamiser pendant 5 minutes sur le tamiseur FRITSCH, avec une amplitude 5 en position permanente,
- peser le refus sur chaque tamis.

Les granules de farine de biomasse de microalgues selon l'invention ne présentent alors aucun refus sur chacun de ces tamis, traduisant un écoulement libre tout à fait conforme à ce qui est obtenu pour les poudres de microalgues riches en protéines de l'état de l'art.

Les granules de farine de biomasse de microalgues selon l'invention sont enfin caractérisés par un degré de mouillabilité satisfaisant, mesuré selon un test B.

La mouillabilité est une propriété technologique très souvent utilisée pour caractériser une poudre remise en suspension dans l'eau, par exemple en Industries Laitières.

Elle traduit l'aptitude d'une poudre à s'immerger après avoir été déposée à la surface de l'eau (Haugaard Sorensen et al., 1978, « Méthodes d'analyse des produits laitiers déshydratés », Niro A/S (ed.), Copenhagen, Denmark), et reflète ainsi la capacité de la poudre à absorber de l'eau à sa surface (Cayot et Lorient, 1998, « Structures et technofonctions des protéines du lait ». Paris : Airlait Recherches: Tec et Doc, Lavoisier).

La détermination de cet indice consiste à mesurer le temps nécessaire à une certaine quantité de poudre pour pénétrer dans l'eau à travers sa surface libre au repos.

Il faut également associer à la mouillabilité, l'aptitude au gonflement de la poudre. En effet, lorsqu'une poudre absorbe de l'eau, elle gonfle progressivement. Puis, la structure de la poudre disparaît lorsque les divers constituants sont solubilisés ou dispersés.

Parmi les facteurs influençant la mouillabilité, il y a la présence de grosses particules primaires, la réintroduction des fines, la masse volumique de la poudre, la porosité et la capillarité des particules de poudre ainsi que la présence d'air, la présence de matières grasses à la surface des particules de poudre et les conditions de reconstitution.

Le test B, mis au point par la société Demanderesse, consiste ici à considérer plus particulièrement le comportement de la poudre de farine de microalgue lors de la mise en contact de l'eau, par la mesure, après un certain temps de contact, de la hauteur de la poudre qui décante lorsque placée à la surface de l'eau.

Le protocole de ce test est le suivant :

- dans un bécher de forme basse de 600 ml (bécher FISCHERBRAND FB 33114), introduire 500 ml d'eau déminéralisée à 20°C,
- placer uniformément 25 g de la poudre de farine de microalgues à la surface de l'eau, sans mélanger,
- observer le comportement de la poudre après 3 h de contact,
- mesurer la hauteur du produit décanté au fond du bécher.

Une poudre très cohésive, collante, de faible mouillabilité demeurera à la surface du liquide, tandis qu'une poudre de meilleure mouillabilité, moins collante, décantera plus aisément.

Les granules de farine de biomasse de microalgues selon l'invention présentent alors un degré de mouillabilité, exprimé selon ce test B, par la hauteur du produit décanté dans un bêcher, à une valeur comprise entre 5 et 25 mm.

Plus particulièrement :

- la première famille présente une hauteur de produit décanté comprise entre 5 et 15 mm,
- la seconde famille présente une hauteur de produit décanté comprise entre 15 et 25 mm.

Les granules de farine de biomasse de microalgues selon l'invention sont également caractérisés par leur surface spécifique.

On détermine la surface spécifique sur l'ensemble de la distribution granulométrique des granules de farine de biomasse de microalgues grâce à un analyseur de surface spécifique Quantachrome basé sur un test d'absorption de l'azote sur la surface du produit soumis à l'analyse, réalisé sur appareil SA3100 de chez Beckmann Coulter, en suivant la technique décrite dans l'article BET Surface Area by Nitrogen Absorption de S. BRUNAUER et al. (Journal of American Chemical Society, 60, 309, 1938).

Les granules de farine de biomasse de microalgues conformes à l'invention, après dégazage 30 minutes à 30°C sous vide, présentent abrs une surface spécifique comprise entre 0,45 et 0,70 $m^2$/g.

Plus particulièrement, la première famille de granules de farine de biomasse de microalgues présente une surface spécifique selon la méthode BET, comprise entre 0,45 et 0,50 $m^2$/g.

Quant à la deuxième famille de granules de farine de biomasse de microalgues, elle présente une surface spécifique selon la méthode BET, comprise entre 0,60 et 0,70 $m^2$/g.

Les granules de farine de biomasse de microalgues de l'invention se différencient des farines de microalgues obtenues par atomisation classique.

Les granules de farine de biomasse de microalgues conformes à l'invention sont susceptibles d'être obtenus par un procédé d'atomisation particulier, qui met en oeuvre des buses de pulvérisation à haute pression dans une tour à flux parallèles qui dirige les particules semi-sèches vers le bas, vers une bande mobile.

La matière est ensuite transportée comme une couche poreuse à travers des zones de post-séchage et de refroidissement, qui lui donnent une structure craquante, comme celle d'un gâteau qui se brise à l'extrémité de la bande et est déchargée, le plus souvent via un système de contrôle final de la taille des particules.

Pour procéder à la granulation de la farine de biomasse d'algues, en suivant ce principe d'atomisation, on peut employer par exemple un atomiseur FILTERMAT™ commercialisé par la société GEA NIRO ou un système de séchage TETRA MAGNA PROLAC DRYER™ commercialisé par la société TETRA PAK.

De manière surprenante et inattendue, la société Demanderesse a ainsi constaté que la granulation de la farine de biomasse de microalgues par la mise en oeuvre par exemple de ce procédé FILTERMAT™, permettait de préparer avec un haut rendement un produit conforme à l'invention sur le plan de la distribution granulométrique, de sa densité aérée et de sa compressibilité.

En effet, les procédés décrits antérieurement (telle l'atomisation simple effet ou multiple effet - tour MSD) ne permettent pas d'obtenir l'ensemble des caractéristiques souhaitées.

Le procédé de préparation des granules de farine de biomasse de microalgues conformes à l'invention comprend alors les étapes suivantes :

1) préparer une suspension de biomasse de microalgues riche en protéines dans de l'eau à une matière sèche comprise entre 10 et 35 % en poids sec,
2) la pulvériser dans un atomiseur vertical équipé d'une bande mobile à sa base, et d'une buse haute pression à sa partie supérieure, tout en réglant :

- la première température de l'air primaire à une valeur comprise entre 160 et 220°C,
- la seconde température de l'air primaire à une valeur comprise entre 90 et 150°C
- la pression de pulvérisation à une valeur comprise entre 50 et 250 bar, de préférence entre 80 et 150 bar,

3) régler la température d'entrée de la zone de post séchage sur la bande mobile à une valeur comprise entre 70 et 90°C, et régler la température de la zone de refroidissement à une valeur comprise entre 15 et 25°C,
4) collecter les granules de farine de biomasse de microalgues ainsi obtenus.

La première étape du procédé de l'invention consiste à préparer une suspension de biomasse de microalgues riche en protéines dans de l'eau à une matière sèche comprise entre 10 et 35 % en poids sec.

Les microalgues choisies pour illustrer le procédé conforme à l'invention sont :

- *Chlorella sorokiniana* (souche UTEX 1663 - *The Culture Collection of Algae at the University of Texas at Austin* - USA),
- *Chlorella protothecoides* (souche UTEX 250 - *The Culture Collection of Algae at the University of Texas at Austin* - USA).

Comme il sera exemplifié ci-après, les biomasses extraites du milieu de fermentation par tout moyen connu de l'homme du métier (par exemple par centrifugation) sont ensuite désactivées par traitement thermique flash.

La deuxième étape du procédé de l'invention consiste à pulvériser la suspension de biomasse dans un atomiseur vertical équipé d'une bande mobile à sa base, et d'une buse haute pression à sa partie supérieure, tout en réglant :

- la première température de l'air primaire à une valeur comprise entre 160 et 220°C,
- la seconde température de l'air primaire à une valeur comprise entre 90 et 150°C
- la pression de pulvérisation à une valeur comprise entre 50 et 250 bar, de préférence entre 80 et 150 bar,

La troisième étape du procédé de l'invention consiste à régler la température d'entrée de la zone de post séchage sur la bande mobile à une valeur comprise entre 70 et 90°C, et régler la température de la zone de refroidissement à une valeur comprise entre 15 et 25°C.

Les granules de farine de biomasse de microalgues présentent, en sortie de chambre principale, une humidité résiduelle comprise entre 8 et 15 %.

Pour amener le degré d'humidité des granules de farine de microalgues à la valeur souhaitée (en sortie de sécheur : entre 3 et 6 %), la société Demanderesse a trouvé qu'il fallait respecter les barèmes de température des zones de séchage et de refroidissement.

La dernière étape du procédé conforme à l'invention consiste enfin à collecter les granules de farine de biomasse de microalgues ainsi obtenus.

Les granules de farine de microalgues conformes à l'invention peuvent être employés, en raison de la qualité de leurs propriétés fonctionnelles mentionnées plus haut, dans les applications en alimentations humaine et animale (animaux de compagnie, aquaculture...), ou dans des applications de l'industrie pharmaceutique et cosmétique. Par industrie pharmaceutique, il est important de noter que les granules de farine de microalgues conformes à l'invention ne sont pas utilisés comme principe actif, mais comme agents de formulation, pour la préparation de comprimés.

Ainsi, la présente invention est également relative à une méthode de préparation de compositions alimentaires humaines ou animales ou de compositions pharmaceutiques ou cosmétiques, comprenant une étape d'incorporation de granules de de farine de microalgues conformes à l'invention.

Il est ainsi trouvé un grand intérêt des granules de farine de microalgues conformes à l'invention en regard de leurs propriétés fonctionnelles :

- leur écoulement libre (poudre moins fine déjà agglomérée) permet de faciliter une alimentation en extrudeur et le remplissage des trémies,
- leur densité aérée plus forte permet de faciliter également leur transport (coût réduit), de réduire l'émission de poussière lors de la manipulation des sacs de poudres ;
- leur dispersion facile dans les liquides permet :

  ◦ d'éviter la formation de grumeaux,
  ◦ de faciliter la préparation pour boisson,
  ◦ de les incorporer dans les milieux visqueux, sans formation de grumeaux ni besoin de « disperseurs » puissants comme les gommes.

- leur aptitude à la compression les destine à la fabrication de granulés solides, insolubles, résistant au procédé de fabrication des aliments qui permettent, pour les granules de farine de microalgues vertes selon l'invention, de créer des produits mouchetés verts attrayants (cake, biscuit, comprimés, gommes, enrobage...).

Par exemple, dans le domaine des compléments alimentaires, les granules de farine de biomasse de microalgues peuvent être aisément incorporés dans des comprimés orodispersibles, formes galéniques adaptées par exemple à la pédiatrie et la gériatrie.

A titre d'illustration, la société Demanderesse a combiné les granules de farine de microalgues selon l'invention à un de ses excipients à désintégration rapide pour la formulation de comprimés orodispersibles : le PEARLITOL® Flash.

Comme il sera exemplifié ci-après, l'évaluation des caractéristiques de ces comprimés montrent que :

- il n'y a pas d'influence négative de l'incorporation des granules de farine de biomasse de microalgues sur la dureté des comprimés ;
- les granules de farine de biomasse de microalgues diminuent la friabilité des comprimés.

D'autres caractéristiques et avantages de l'invention apparaîtront à la lecture des exemples qui suivent. Ils ne sont toutefois donnés ici qu'à titre illustratif et non limitatif.

## EXEMPLES

### Exemple 1 : Production de *C. sorokiniana* en fermentation de type « fed batch » avec apport en glucose limitant

La souche utilisée est une *Chlorella sorokiniana* (souche UTEX 1663 - *The Culture Collection of Algae at the University of Texas at Austin* - USA).

*Préculture :*

- 600 mL de milieu dans un Erlenmeyer de 2 L ;
- Composition du milieu (tableau 1 ci-dessous)

Tableau 1.

| | | | |
|---|---|---|---|
| Macro éléments (g/L) | Glucose | | 20 |
| | $K_2HPO_4.3H_2O$ | | 0,7 |
| | $MgSO_4.7H_2O$ | | 0,34 |
| | Acide citrique | | 1,0 |
| | Urée | | 1,08 |
| | $Na_2SO_4$ | | 0,2 |
| | $Na_2CO_3$ | | 0,1 |
| | Extrait de levure | | 1 |
| | clerol FBA 3107 (antimousse) | | 0,5 |
| Micro éléments (mg/L) | $Na_2EDTA$ | | 10 |
| | $CaCl_2.2H_2O$ | | 80 |
| | $FeSO_4.7H_2O$ | | 40 |
| | $MnSO_4.4H_2O$ | | 0,41 |
| | $CoSO_4.7H_2O$ | | 0,24 |
| | $CuSO_4.5H_2O$ | | 0,24 |
| | $ZnSO_4.7H_2O$ | | 0,5 |
| | $H_3BO_3$ | | 0,11 |
| | $(NH_4)_6Mo_7O_{27}.4H_2O$ | | 0,04 |

Le pH est ajusté à 7 avant stérilisation par ajout de NaOH 8N.

L'incubation se déroule dans les conditions suivantes :

- durée : 72 h ;
- température : 28°C ;
- agitation : 110 rpm (Incubateur Infors Multitron).

La préculture est ensuite transférée dans un fermenteur de 30L de type Sartorius.

*Culture pour production de biomasse :*

Le milieu de départ est similaire à celui de la préculture :

Tableau 2.

| | | |
|---|---|---:|
| Macro éléments (g/L) | Glucose | 10 |
| | KH$_2$PO$_4$ | 8 |
| | MgSO$_4$.7H2O | 0,35 |
| | Ac Citrique | 1,0 |
| | (NH$_4$)$_2$SO$_4$ | 0,2 |
| | Na$_2$SO$_4$ | 1 |
| | Extrait de levure | 3,2 |
| | clerol FBA 3107 (antimousse) | 0,2 |
| Micro éléments (mg/L) | Na$_2$EDTA | 10 |
| | CaCl$_2$ | 100 |
| | FeSO$_4$.7H2O | 400 |
| | MnSO$_4$.4H2O | 4,1 |
| | CoSO$_4$.7H2O | 2,4 |
| | CuSO$_4$.5H2O | 2,4 |
| | ZnSO$_4$.7H2O | 5 |
| | H$_3$BO$_3$ | 1,1 |
| | (NH$_4$)$_6$Mo$_7$O$_{27}$.4H$_2$O | 0,4 |

Le volume initial (Vi) du fermenteur est ajusté à 13,5 L après ensemencement. Il est porté à 16 - 20 L en final.

Les paramètres de conduite de la fermentation sont les suivants :

Tableau 3.

| | |
|---|---|
| Température | 28 °C |
| pH | 6,5 par NH$_3$ 28% p/p |
| pO$_2$ | > 20% (maintenue par agitation) |
| Agitation | 300 RPM mini |
| Débit d'air | 15 L/min |

Lorsque le glucose apporté initialement est consommé, un apport de milieu est réalisé en continu sous forme d'une solution concentrée contenant 500 g/L de glucose et 8 g/L de MgSO$_4$.7H$_2$O.

La vitesse d'apport est inférieure à la vitesse de consommation que la souche pourrait réaliser de sorte que la teneur résiduelle en glucose dans le milieu est maintenue nulle, c'est-à-dire que la croissance de la souche est limitée par la disponibilité en glucose (condition glucose-limitant).

De l'antimousse Clerol FBA 3107 est ajouté à la demande pour éviter un moussage excessif.

Résultats :

Après 75 h de culture, on obtient 74 g/L de biomasse à une teneur en protéines (évalué par le N 6,25) de 57 %.

Pour la suite des opérations, la température est maintenue sous 8-10°C.

Après cette étape, la concentration de la biomasse est d'environ 18 % (masse cellulaire sèche).

**Exemple 2. Séchage de la biomasse de *Chlorella sorokiniana***

On procède au séchage de la biomasse obtenue à l'exemple 1 :

- dans un dispositif FILTERMAT, pour obtenir les granules de farine de microalgues conformes à l'invention,

- dans un atomiseur multiple effet (liquide séché dans le flux de chaleur puis récupéré en bas de la tour au niveau du cyclone ou du filtre à manche), de manière à obtenir une farine de microalgues témoin, conforme à ce qui est commercialement accessible.

Les principales conditions opératoires d'atomisation multiple effet d'une suspension de biomasse de microalgues à 18 % de matière sèche sont les suivantes :

- pression de pulvérisation (2 buses) : 150 bars
- température d'entrée : 275°C,
- température de sortie : 80°C.
- température du lit statique : 80°C
- refroidissement sur lit fluidisé vibré :

  ◦ température entrée 1ère section : 50°C
  ◦ température entrée 2ème section : 20°C

Quant au procédé d'atomisation conforme à l'invention, il consiste à pulvériser la biomasse à haute pression dans un dispositif de type FILTERMAT commercialisé par la société GEA/NIRO, muni d'une buse d'injection haute pression de type DELAVAN, dans les conditions suivantes :

- matière sèche de la suspension de biomasse de microalgues : 18 %
- première température de l'air primaire : 175°C +/-10°C
- seconde température de l'air primaire : 110°C +/-10°C
- pression de pulvérisation : 120 bar
- température d'entrée de la zone de post séchage : 80°C
- température de la zone de refroidissement : 18°C
- température de sortie de la chambre : 55°C +/- 2°C

Après séchage, les granules de farine de biomasse de microalgues présentent ne humidité résiduelle comprise entre 3 et 6 %.

**Exemple 3 : Production de *Chlorella protothecoides* à 28°C en fermentation de type fed-batch**

Pour obtenir une concentration en biomasse élevée, nous apportons le glucose en cours de culture (fed-batch) pour éviter une inhibition de la croissance par le glucose.
Les apports en sels sont effectués au départ de la fermentation (Batch).
La souche utilisée est *Chlorella protothecoides UTEX 250 (The Culture Collection of Algae at the University of Texas at Austin* - USA).

Pré-culture :

- 500 mL de milieu dans un Erlenmeyer de 2L ;
- Composition du milieu (en g/L) :

Tableau 4.

| | | | |
|---|---|---|---|
| Macro éléments (g/L) | | Glucose | 40 |
| | | $K_2HPO_4$ | 3 |
| | | $Na_2HPO_4$ | 3 |
| | | $MgSO_4.7H_2O$ | 0,25 |
| | | $(NH_4)_2SO_4$ | 1 |
| | | Ac Citrique | 1 |
| | | clerol FBA 3107 (antimousse) | 0,1 |
| éléments et Vitamines | | $CaCl_2.2H_2O$ | 30 |
| | | $FeSO_4.7H_2O$ | 1 |
| | | $MnSO_4.1H_2O$ | 8 |

| | |
|---|---|
| $CoSO_4.7H_2O$ | 0,1 |
| $CuSO_4.5H_2O$ | 0,2 |
| $ZnSO_4.7H_2O$ | 0,5 |
| $H_3BO_3$ | 0,1 |
| $Na_2MoO_4 \cdot 2H_2O$ | 0,4 |
| Thiamine HCl | 1 |
| Biotine | 0,015 |
| B12 | 0,01 |
| Calcium pantothénate | 0,03 |
| acide p-aminobenzoïque | 0,06 |

L'incubation se déroule dans les conditions suivantes : durée : 72 h ; température : 28°C ; agitation : 110 rpm (Incubateur Infors Multtron).
La pré-culture est ensuite transférée dans un fermenteur de 30L de type Sartorius.

Culture pour production de biomasse :

Le milieu est le suivant :

Tableau 5.

| | | | |
|---|---|---|---|
| Macro éléments (g/L) | Glucose | 40 |
| | KH$_2$PO$_4$ | 1,8 |
| | NaH$_2$PO$_4$ | 1,4 |
| | MgSO$_4$.7H$_2$O | 3,4 |
| | (NH$_4$)$_2$SO$_4$ | 0,2 |
| | clerol FBA 3107 (antimousse) | 0,3 |
| Micro-éléments et Vitamines (mg/L) | CaCl$_2$.2H$_2$O | 40 |
| | FeSO$_4$.7H$_2$O | 12 |
| | MnSO$_4$.1H$_2$O | 40 |
| | CoSO$_4$.7H$_2$O | 0,1 |
| | CuSO$_4$.5H$_2$O | 0,5 |
| | ZnSO$_4$.7H$_2$O | 50 |
| | H$_3$BO$_3$ | 15 |
| | Na$_2$MoO$_4$ · 2H$_2$O | 2 |
| | Thiamine HCl | 6 |
| | Biotine | 0,1 |
| | B12 | 0,06 |
| | Calcium pantothénate | 0,2 |
| | Acide p-aminobenzoïque | 0,2 |

Le volume initial (Vi) du fermenteur est ajusté à 17 L après ensemencement. Il est porté à 20 à 25 L environ en final.

Les paramètres de conduite de la fermentation sont les suivants :

Tableau 6.

| | |
|---|---|
| Température | 28 °C |
| pH | 5,0 - 5,2 par NH$_3$ 28% p/p |
| pO$_2$ | 20% +/- 5% (maintenue par agitation) |
| Agitation | 300 RPM mini |
| Débit d'air | 15 L/min |

Lorsque la concentration résiduelle en glucose tombe en dessous de 10 g/L, un apport de glucose sous forme d'une solution concentrée à 800 g/L environ est réalisé de façon à maintenir la teneur en glucose entre 0 et 20 g/L dans le fermenteur.

Résultats

En 40h, nous obtenons 89 g/L de biomasse contenant 68,5 % de protéines.

Les cellules sont désactivées par traitement thermique à travers une zone HTST à 70°C pendant 3 minutes

Pour la suite des opérations, la température est maintenue sous 8-10°C.

Après cette étape, la concentration de la biomasse est d'environ 20 % (masse cellulaire sèche).

**Exemple 4 : Séchage de la biomasse de *Chlorella protothecoides***

On procède au séchage de la biomasse obtenue à l'exemple 3 :

- dans un dispositif FILTERMAT, pour obtenir les granules de farine de microalgues conformes à l'invention,

- dans un atomiseur multiple effet (liquide séché dans le flux de chaleur puis récupéré en bas de la tour au niveau du cyclone ou du filtre à manche), de manière à obtenir une farine de microalgues témoin, conforme à ce qui est commercialement accessible.

Les principales conditions opératoires d'atomisation multiple effet d'une suspension de biomasse de microalgues à 20 % de matière sèche sont les suivantes :

- pression de pulvérisation (2 buses) : 150 bars

- température d'entrée : 270°C,

- température de sortie : 80°C.

- température du lit statique : 80°C

- refroidissement sur lit fluidisé vibré :

    ◦ température entrée 1ère section : 50°C

    ◦ température entrée 2ème section : 20°C

Quant au procédé d'atomisation conforme à l'invention, il consiste à pulvériser la biomasse à haute pression dans un dispositif de type FILTERMAT commercialisé par la société GEA/NIRO, muni d'une buse d'injection haute pression de type DELAVAN, dans les conditions suivantes :

- matière sèche de la suspension de biomasse de microalgues : 20 %

- première température de l'air primaire : 174°C +/-10°C

- seconde température de l'air primaire : 102°C +/-10°C

- pression de pulvérisation : 150 bar

- température d'entrée de la zone de post séchage : 80°C

- température de la zone de refroidissement : 20°C

- température de sortie de la chambre : 57°C +/- 3°C

Après séchage, les granules de farine de biomasse de microalgues présentent une humidité résiduelle comprise entre 3 et 6 %.

**Exemple 5. Caractérisation des granules de farine de biomasse de microalgues conformes à l'invention**

Le Tableau 7 suivant présente le profil physicochimique de 4 lots de granules de farine de microalgues selon l'invention (deux lots produits avec *Chlorella sorokiniana* - Lots 1 et 2 - et deux lots avec *Chlorella protothecoides* - Lots 3 et 4), en comparaison avec des farines séchées sur atomiseur multiple effet (sur une tour MSD).

Tableau 7.

| | | *Chlorella sorokiniana* | | | *Chlorella protothecoides* | | |
|---|---|---|---|---|---|---|---|
| | | Lot 1 selon l'invention | Lot 2 selon l'invention | Témoin Atomisation MSD | Lot 3 selon l'invention | Lot 4 selon l'invention | Témoin Atomisation MSD |
| Matière sèche | % | 92,2 | 91 | 95,2 | 92,8 | 93,2 | 97,3 |

(suite)

|  | | Chlorella sorokiniana | | | Chlorella protothecoides | | |
|---|---|---|---|---|---|---|---|
|  | | Lot 1 selon l'invention | Lot 2 selon l'invention | Témoin Atomisation MSD | Lot 3 selon l'invention | Lot 4 selon l'invention | Témoin Atomisation MSD |
| Matière protéique Nx6.25 | % | 54,3 | 54,5 | 48,7 | 62,8 | 62,6 | 60,8 |
| Amidon | % | 6,9 | 5,3 | 18,3 | 6,8 | 6,4 | 5,0 |
| Lipides Totaux | % | 9,3 | 8,9 | 8,2 | 10,7 | 12,7 | 14,7 |
| Chlorophylles totaux | % | 2,49 | 2,15 | 1,97 | <0,05 | <0,05 | Nd |
| Caroténoïdes totaux | % | 0,47 | 0,46 | 0,39 | <0,05 | <0,05 | Nd |
| Sucres totaux | % | 16,6 | 15,4 | 30,1 | 21,3 | 17,9 | 26,1 |
| *nd : non détecté | | | | | | | |

Sont notamment présentées dans le tableau 8 suivant les valeurs des paramètres de :

- granulométrie,

- compressibilité,

- densité apparente,

- surface spécifique,

- écoulement,

- mouillabilité

des granules de farine de biomasse de microalgues conformes à l'invention, comparativement à ces mêmes paramètres d'une farine de microalgues séchées par atomisation classique.

Tableau 8.

|  | | Chlorella sorokiniana | | | Chlorella protothecoides | | |
|---|---|---|---|---|---|---|---|
|  | | Lot 1 selon l'invention | Lot 2 selon l'invention | Témoin Atomisation MSD | Lot 3 selon l'invention | Lot 4 selon l'invention | Témoin Atomisation MSD |
| Granulométrie laser | Dmode $\mu$m | 127,6 | 72,9 | 203,5 | 269,2 | 223,4 | 223,4 |
| | D4,3 $\mu$m | 129,4 | 79,3 | 210,1 | 404,8 | 333,6 | 227,8 |
| Surface spécifique | m²/g | 0,45 | 0,48 | 0,5 | 0,62 | 0,65 | Nd |
| densité aérée | g/ml | **0,64** | **0,65** | **0,47** | **0,61** | **0,61** | 0,37 |
| densité tassée | g/ml | 0,79 | 0,82 | 0,53 | 0,75 | 0,75 | 0,43 |
| compressibilité | % | **19** | **20,7** | **11,3** | **18,7** | **18,7** | **14** |

(suite)

| | | Chlorella sorokiniana | | | Chlorella protothecoides | | |
|---|---|---|---|---|---|---|---|
| | | Lot 1 selon l'invention | Lot 2 selon l'invention | Témoin Atomisation MSD | Lot 3 selon l'invention | Lot 4 selon l'invention | Témoin Atomisation MSD |
| cohésion 2000 μm | μm | 0 | 0 | 0 | 0 | 0 | 0 |
| cohésion 1400 μm | μm | 0 | 0 | 0 | 0 | 0 | 0 |
| cohésion 800 μm | μm | 0 | 0 | 0 | trace | trace | 0 |
| mouillabilité | mm | 5* | 15** | 20*** | 20* | 20 ** | 20*** |

*Pour la mesure de la mouillabilité :*
*Chlorella sorokiniana :*
*(\*) à l'introduction de la poudre dans le bécher, le produit migre lentement vers le fond - à T3h : 5 mm de dépôt et à peu près 50% de produit à la surface*
*(\*\*) à l'introduction de la poudre dans le bécher, le produit migre lentement vers le fond - à T3h : 15 mm de dépôt et à peu prés 20% de produit à la surface*
*(\*\*\*) le produit tombe directement au fond du bécher au moment du dépôt de la poudre à la surface de l'eau.*
*Chlorella protothecoides*
*(\*) le produit tombe instantanément au fond du bécher, en un bloc*
*(\*\*) à T.3h la totalité du produit s'est déposée dans le fond*
*(\*\*\*) même observation que pour le premier essai sauf qu'à T3h seulement une partie de la poudre a migré vers le fond et environ 20 % du produit reste en surface*

**Exemple 6. Incorporation des granules de farine de biomasse de microalgues dans des comprimés orodispersibles.**

Dans cet exemple, on réalise des comprimés orodispersibles combinant les granules de farine de biomasse de *Chlorella sorokiniana* (Lot 1 de l'exemple 5) avec du PEARLITOL® Flash (amidon et mannitol granulés) commercialisé par la société Demanderesse.

La fabrication des comprimés repose sur les paramètres suivants :

➢ Résistance maxi des poinçons plats chanfreinés de diamètre 13mm = 92 Kn.

➢ poinçon de diamètre 13mm présentant une surface (section) de 1,327 cm$^2$.

On applique 5 forces de compression différentes (exprimées en « force de poinçon supérieur ») - de 5, 10, 15, 20 et 25 kN - sur la même poudre, afin d'obtenir des comprimés de 5 duretés croissantes (essais référencées 1 à 5 dans les tableaux ci-dessous).

Deux formules de comprimés sont élaborées avec (de l'ordre de 10 %) ou sans granules de farine de microalgues selon l'invention, et les paramètres de dureté et de texture sont évalués.

***Comprimés contenant des granules de farine de biomasse de microalgues***

*Formule:*

| | | |
|---|---|---|
| PEARLITOL® Flash | 89,7 % | 627,9 g |
| Lot 1 | 10,0 % | 70,0 g |
| Stéarate de magnésium Bärlocher végétal | 0,3 % | 2,1 g |

*Mode opératoire:*

➢ Dans un récipient de deux litres, introduire la totalité du PEARLITOL® Flash et du Lot 1, puis mélanger pendant cinq minutes à l'aide d'un mélangeur TURBULA®.

➢ Ajouter le stéarate de magnésium puis mélanger à nouveau à l'aide du TURBULA® pendant cinq minutes.

➢ Compression du mélange sur une presse à comprimés KORSCH XP1 équipée de poinçons plats de diamètre 13 mm à la cadence 20 comprimés / min.

Tableau 9.

| Comprimés préparés selon 5 différentes forces de compression | 1 | 2 | 3 | 4 | 5 |
|---|---|---|---|---|---|
| Force poinçon sup. (kN) | 5,033 | 10,025 | 15,376 | 20,056 | 25,470 |
| Ecart type poinçon sup (kN) | 0,026 | 0,053 | 0,088 | 0,145 | 0,126 |
| Force poinçon inf. (kN) | 4,695 | 9,250 | 14,119 | 18,398 | 23,501 |
| Ecart type poinçon inf (kN) | 0,022 | 0,047 | 0,079 | 0,135 | 0,122 |
| Force éjection (N) | 96,777 | 201,465 | 258,040 | 299,089 | 306,315 |
| Ecart type force éjection (N) | 21,645 | 7,015 | 10,558 | 11,758 | 4,869 |
| Déplacement poinçon sup (mm) | 7,602 | 8,075 | 8,357 | 8,572 | 8,730 |
| Déplacement poinçon inf. (mm) | 8,036 | 8,041 | 8,050 | 8,052 | 8,059 |
| Transmission (%) | 93,275 | 92,272 | 91,828 | 91,735 | 92,271 |
| Ecart type de transmission. (%) | 0,398 | 0,117 | 0,184 | 0,074 | 0,088 |
| Poids comprimés (mg) | 601,2 | 600,8 | 602,6 | 602,0 | 604,9 |
| Ecart type poids (mg) | 0,8 | 1,0 | 1,0 | 1,6 | 1,0 |
| Epaisseur comprimés (mm) | 4,45 | 4,05 | 3,84 | 3,67 | 3,59 |
| Ecart type épaisseur (mm) | 0,02 | 0,02 | 0,04 | 0,02 | 0,02 |
| Densité des comprimés | 1,018 | 1,109 | 1,182 | 1,236 | 1,269 |
| Dureté Schleuniger (N) | 0,0 | 23,2 | 54,5 | 81,5 | 106,9 |
| Ecart type dureté (N) | 0,0 | 0,8 | 1,4 | 1,0 | 2,3 |
| Friabilité comprimés (%) | 100 | 100 | 0,22 | 0,16 | 0,11 |
| Temps délitement comp. (s) | 138 | 148 | 147 | 156 | 158 |
| Ecart type délitement (s) | 6 | 12 | 12 | 6 | 12 |

**Comprimés "témoins"**

*Formule:*

| PEARLITOL® Flash lot E019F | 99,7 % | 627,9 g |
|---|---|---|
| Stéarate de magnésium Bärlocher végétal | 0,3 % | 2,1 g |

*Mode opératoire:*

Dans un récipient de deux litres, introduire le PEARLITOL® Flash et le stéarate de magnésium puis mélanger à l'aide du mélangeur TURBULA® pendant cinq minutes.

Compression du mélange sur une presse à comprimés KORSCH XP1 équipée de poinçons plats de diamètre 13 mm à la cadence 20 comprimés / min.

Tableau 10.

| Comprimés préparés selon 5 différentes forces de compression | 1 | 2 | 3 | 4 | 5 |
|---|---|---|---|---|---|
| Force poinçon sup. (kN) | 5,098 | 10,215 | 15,054 | 20,206 | 25,117 |
| Ecart type poinçon sup (kN) | 0,025 | 0,035 | 0,075 | 0,081 | 0,069 |
| Force poinçon inf. (kN) | 4,845 | 9,600 | 14,109 | 18,918 | 23,550 |
| Ecart type poinçon inf (kN) | 0,024 | 0,030 | 0,061 | 0,070 | 0,058 |
| Force éjection (N) | 102,734 | 173,730 | 226,367 | 270,345 | 294,141 |
| Ecart type force éjection (N) | 3,316 | 10,527 | 10,472 | 13,976 | 9,736 |
| Déplacement poinçon sup (mm) | 7,864 | 8,293 | 8,561 | 8,732 | 8,894 |
| Déplacement poinçon inf. (mm) | 8,255 | 8,217 | 8,207 | 8,188 | 8,189 |
| Transmission (%) | 95,036 | 93,978 | 93,724 | 93,629 | 93,760 |
| Poids comprimés (mg) | 603,0 | 602,4 | 601,7 | 603,4 | 600,6 |
| Ecart type poids (mg) | 0,4 | 0,8 | 0,7 | 0,7 | 0,9 |
| Epaisseur comprimés (mm) | 4,37 | 3,95 | 3,75 | 3,62 | 3,51 |
| Ecart type épaisseur (mm) | 0,01 | 0,01 | 0,01 | 0,02 | 0,01 |
| Densité des comprimés | 1,040 | 1,149 | 1,206 | 1,256 | 1,289 |
| Dureté Schleuniger (N) | 0,0 | 27,3 | 58,6 | 91,8 | 116,9 |
| Ecart type dureté (N) | 0,0 | 0,7 | 0,8 | 1,3 | 1,7 |
| Friabilité comprimés (%) | 100 | 100 | 0,95 | 0,25 | 0,05 |
| Temps délitement comp. (s) | 58 | 61 | 78 | 82 | 92 |
| Ecart type délitement (s) | 6 | 4 | 7 | 8 | 9 |
| Ecart type de transmission. (%) | 0,325 | 0,276 | 0,165 | 0,099 | 0,092 |

**Conclusions** :

Deux différentes poudres ont été comprimées à l'aide de la presse alternative KORSCH équipée de poinçons plats de diamètre 13 mm :

- En témoin, une poudre avec PEARLITOL® Flash seul,

- une autre avec 90% PEARLITOL® Flash et 10% de granules de farine de microalgues conformes à l'invention (Lot 1).

Le taux de lubrifiant est constant pour les deux formules avec 0,3 % de stéarate de magnésium.
A la lecture des résultats, il apparaît :

- que tous les mélanges sont homogènes et s'écoulent parfaitement. De même, il n'y a aucun problème de collage et/ou de clivage.

- qu'il n'y a pas d'influence négative de la composante « granules de farine de microalgues » sur la dureté des comprimés.

- les granules de farine de microalgues selon l'invention diminuent la friabilité des comprimés.

**Exemple 7. Formulation des granules de farine de biomasse de microalgues en compléments alimentaires**

11 recettes ont été élaborées à partir des granules du Lot 1.

*Soupe aux poireaux*

| | Témoin | Essai |
|---|---|---|
| Lait entier poudre | 33 | 33 |
| NUTRIOSE® FB06 | 8 | 8 |
| GLUCIDEX® IT21 | 7 | 7 |
| Bouillon de volaille | 5 | 5 |
| Poireaux en poudre | 4 | 4 |
| PREGEFLO® CH10 | 3 | 3 |
| Lot 1 | 0 | 1,5 |
| Oignons en poudre | 2,5 | 2,5 |
| Sel | 0,5 | 0,5 |
| Ail en poudre | 0,5 | 0,5 |
| Poivre | 0,1 | 0,1 |
| Persil déshydraté | 0,3 | 0,3 |
| Total : | 63,9 | 65,4 |
| Eau | 250 | 250 |

*Prémélanger toutes les poudres ensemble*
*Disperser dans l'eau chaude et mixer*

*Soupe aux légumes (glutamates)*

| Lait entier poudre | 33 |
|---|---|
| NUTRIOSE® FB06 | 8 |
| GLUCIDEX® IT21 | 7 |
| Bouillon de volaille | 2,5 |
| Poireaux en poudre | 0,5 |
| Epinards poudre | 3 |
| Tomates poudre | 1,5 |
| Carottes poudre | 1 |
| PREGEFLO® CH10 | 3 |
| Lot 1 | **1,5** |
| Oignons en poudre | 1,4 |
| Oignons grillés | 1,1 |
| Sel | 0,5 |
| Ail en poudre | 0,6 |
| Poivre | 0 |
| Glutamate | 0,2 |

| Muscade | 0,3 |
|---|---|
| Persil poudre | 0,3 |
| Total : | **65,4** |
| Eau | 250 |

*Prémélanger toutes les poudres ensemble*
*Disperser dans l'eau chaude et mixer*

*Cake au citron riche en fibres*

| | | | Témoin | Essai | Témoin | Essai |
|---|---|---|---|---|---|---|
| **A** | | Beurre pommadé 82% MGr | 200 | 200 | 19,31 | 19,31 |
| | | Sucre poudre | 252 | 217 | 24,32 | 20,95 |
| | | Colorant jaune d'œuf | 1 | 1 | 0,10 | 0,10 |
| | | Arome citron Mane | 0 | 4 | 0 | 0,39 |
| | | Arome vanille | 4 | 0 | 0,39 | 0 |
| | | Sel | 3 | 3 | 0,29 | 0,29 |
| **B** | | Œuf entire | 180 | 180 | 17,37 | 17,37 |
| | | Lait UHT 1/2 écrémé | 100 | 100 | 9,65 | 9,65 |
| **C** | | Farine Leforest | 178 | 153 | 17,18 | 14,77 |
| | | Fécule de PdT | 70 | 70 | 6,76 | 6,76 |
| | | Fibre de pois W8024 | 0 | 25 | 0 | 2,41 |
| | | NUTRIOSE® FB06 | 0 | 35 | 0 | 3,38 |
| | | Spongolit 283 | 6 | 6 | 0,58 | 0,58 |
| | | Poudre levante, volcano | 6 | 6 | 0,58 | 0,58 |
| **D** | | Lot 1 | **0** | **36** | **0** | **3,47** |
| | | Pépites de chocolat | 36 | 0 | 3,47 | 0 |
| | | Total : | **1036** | **1036** | **100** | **100** |

*Dans le bol hobart à l'aide de la feuille, mélanger* A *30 sec V1 puis 2 min V2*
*Incorporer* B *mélanger 1 min V1 muis 2 min V2*
*Ajouter* C *mélanger 1 min V1 puis 3 min V2*
*Incorporer* D *mélanger 15 sec V1*
*Remplir les moules et cuisson*
*Cuire en four rotatif 18min à 170°C*
*Quantité pour 32 cakes de 31g environ*
*Perte en eau à la cuisson : 15%*
*Lot 1 avec 10% de NUTRIOSE® FB06*

*Biscuits*

| | | Témoin *100% Sucre* | Essai *100% MALTISORB®* | Témoin *100% Sucre* | Essai *100% MALTISORB®* |
|---|---|---|---|---|---|
| **A** | Eau | 85 | 95 | 7,83 | 8,68 |
| | Bicarbonate de soude | 3 | 3 | 0,28 | 0,27 |
| | Bicarbonate d'ammonium | 2 | 2 | 0,18 | 0,18 |
| **B** | Sucre glace | 180 | 0 | 16,59 | 0 |
| | Maltisorb P200 | 0 | 180 | 0 | 16,44 |
| **C** | MGr, Biscuitine 500 | 160 | 160 | 14,75 | 14,61 |
| | Lécithine de soja | 2 | 2 | 0,18 | 0,18 |
| **D** | Farine Leforest | 637 | 637 | 58,71 | 58,17 |
| | Lot 1 | **10** | **10** | **0,92** | **0,91** |
| | Sel | 2 | 2 | 0,18 | 0,18 |
| | Arôme vanille | 4 | 4 | 0,37 | 0,37 |
| | *Total :* | **1085** | **1095** | **100** | **100** |

*Dans le bol hobart à l'aide de la feuille, mélanger* A *2 min V1*
*Incorporer* B *mélanger 1 min V1, puis 2 min V2*
*Ajouter* C *mélanger 1 min V1, puis 3 min V2*
*Introduire le mélange des poudres* C, *mélanger 4 min V1*
*Laisser reposer la pâte 15 minutes*
*La passer dans la rotative à biscuits*
*Déposer les biscuits sur une plaque et cuire*

| | Témoin | Essai |
|---|---|---|
| *Four rotatif 125°c* | 30 min | |
| *Coloration* | **++** | |
| *Four rotatif 150°c* | 15 min | |
| *Coloration* | **++++** | |
| *Four rotatif 170°c* | 9 min | |
| *Coloration* | **++++++** | **0** |

*Ketchup*

| | Essai 1 | Essai 2 | Essai 3 | Essai 4 | Essai 5 | Essai 6 | Essai 7 |
|---|---|---|---|---|---|---|---|
| | Sans arôme | Arôme Ketchup | Arôme Tomate | Arôme Heinz | Arôme Tomate Giv. | Arôme Ketchup + Tomate (Mane) | Arôme Heinz + Tomate Giv. |
| Pea Starch | 5 | 5 | 5 | 5 | 5 | 5 | 5 |
| CLEARAM® CH2020 | 25 | 25 | 25 | 25 | 25 | 25 | 25 |
| MALTISORB® 75/75 | 66 | 66 | 66 | 66 | 66 | 66 | 66 |
| NUTRIOSE® FB17 | 60 | 60 | 60 | 60 | 60 | 60 | 60 |
| Vinaigre 8° | 50 | 50 | 50 | 50 | 50 | 50 | 50 |
| Sel | 10 | 10 | 10 | 10 | 10 | 10 | 10 |
| Eau | 281 | 281 | 280 | 281 | 275 | 281 | 278 |
| Arôme Ketchup Mane | 0 | 0,25 | 0 | 0 | 0 | 0,125 | 0 |
| Arôme Tomate Mane | 0 | 0 | 0,75 | 0 | 0 | 0,375 | 0 |
| Arôme Ketchup Heinz | 0 | 0 | 0 | 0,35 | 0 | 0 | 0,175 |
| Arôme Tomate Givaudan | 0 | 0 | 0 | 0 | 6 | 0 | 3 |
| Lot 1 | 2,5 | 2,5 | 2,5 | 2,5 | 2,5 | 2,5 | 2,5 |
| Acesulfame K | 0,3 | 0,25 | 0,25 | 0,25 | 0,25 | 0,25 | 0,25 |
| Total : | 499,8 | 500 | 499,5 | 500,1 | 499,75 | 500,25 | 499,925 |

*Mixer tous les ingrédients ensemble*

*Cuire au bain-marie pendant 10 min à 90-95°C*

| | Essai 1 | Essai 2 | Essai 3 | Essai 4 | Essai 5 | Essai 6 | Essai 7 |
|---|---|---|---|---|---|---|---|
| | Sans arôme | Arôme Ketchup | Arôme Tomate | Arôme Heinz | Arôme Tomate Giv. | Arôme Ketchup + Tomate (Mane) | Arôme Heinz + Tomate Giv. |
| Amidon de pois | 1,00 | 1,00 | 1,00 | 1,00 | 1,00 | 1,00 | 1,00 |
| CLEARAM® CH2020 | 5,00 | 5,00 | 5,01 | 5,00 | 5,00 | 5,00 | 5,00 |
| MALTISORB® 75/75 | 13,21 | 13,20 | 13,21 | 13,20 | 13,21 | 13,19 | 13,20 |
| NUTRIOSE® FB17 | 12,00 | 12,00 | 12,01 | 12,00 | 12,01 | 11,99 | 12,00 |
| Vinaigre 8° | 10,00 | 10,00 | 10,01 | 10,00 | 10,01 | 10,00 | 10,00 |
| Sel | 2,00 | 2,00 | 2,00 | 2,00 | 2,00 | 2,00 | 2,00 |
| Eau | 56,22 | 56,20 | 56,06 | 56,19 | 55,03 | 56,17 | 55,61 |
| Arôme Ketchup Mane | 0 | 0,05 | 0 | 0 | 0 | 0,02 | 0 |
| Arôme Tomate Mane | 0 | 0 | 0,15 | 0 | 0 | 0,07 | 0 |
| Arôme Ketchup Heinz | 0 | 0 | 0 | 0,07 | 0 | 0 | 0,04 |
| Arôme Tomate Givaudan | 0 | 0 | 0 | 0 | 1,20 | 0 | 0,60 |
| Lot 1 | 0,50 | 0,50 | 0,50 | 0,50 | 0,50 | 0,50 | 0,50 |
| Acesulfame K | 0,06 | 0,05 | 0,05 | 0,05 | 0,05 | 0,05 | 0,05 |
| Total : | 100 | 100 | 100 | 100 | 100 | 100 | 100 |

*Mayonnaise*

|  |  | Témoin | Essai | Témoin | Essai |
|---|---|---|---|---|---|
| A | Sucre Poudre | 50 | 50 | 5,00 | 4,98 |
|  | Vinaigre 8° | 100 | 100 | 10,00 | 9,95 |
|  | Moutarde | 40 | 40 | 4,00 | 3,98 |
|  | Sel | 30 | 30 | 3,00 | 2,99 |
|  | Eau | 393 | 393 | 39,30 | 39,10 |
|  | Colorant Jaune d'Œuf | 1,5 | 1,5 | 0,15 | 0,15 |
|  | Lot 1 | 0 | 5 | 0 | 0,50 |
|  | Sorbate de Potassium (E202) | 0,5 | 0,5 | 0,05 | 0,05 |
| B | Jaune d'œufs frais | 50 | 50 | 5,00 | 4,98 |
| C | Huile de Colza | 300 | 300 | 30,00 | 29,85 |
|  | PREGEFLO® PJ30 | 35 | 35 | 3,50 | 3,48 |
| | Total : | 1000 | 1005 | 100 | 100 |

*Dans un bol mixer, mélanger A pendant 1 min*
*Incorporer le jaune d'oeufs (B)*
*Ajouter C en filet, mélanger à vitesse maxi*
*Continuer l'agitation pendant 1 minute*

*"Wine gums"*

|  | Essai |
|---|---|
| FLOKYS® B6080S | 45,5 |
| CLEARGUM® LG7015 | 10,8 |
| Pectine PG769S | 0,5 |
| Saccharose | 26,3 |
| Lot 1 | 1,0 |
| Eau | 14,2 |
| Acide citrique 50% | 2,8 |
| Total : | 101,0 |

*Cacahuètes enrobées*

|  | Essai |
|---|---|
| Cacahuètes | 27,3 |
| Nutriose FB 06 | 34,4 |
| Lot 1 | **3** |
| PREGEFLO® CH10G | 25,3 |
| Farine | 6,2 |
| Amidon de maïs | 3,8 |
| Total : | **100** |

Chauffer au bain-marie le sirop de NUTRIOSE® FB 06 (40% MS) à 50°C

Mélanger les poudres

Mettre les cacahuètes grillées dans la turbine

Ajouter le sirop de NUTRIOSE® FB 06 (15-20g) et le mélange des poudres (30g environ)

Répéter cette opération autant de fois que possible

Cuire au four à 200°C pendant 7 minutes

Sauce Pesto

|  | Témoin | Essai 1 | Essai 2 |
|---|---|---|---|
| Basilic | 33,0 | 24,0 | 20,0 |
| Fromage | 5,2 | 7,2 | 5,0 |
| Sel | 1,5 | 1,5 | 1,5 |
| Acide lactique | 0,7 | 0 | 0 |
| Pignon | 2,1 | 2,1 | 0,5 |
| Ail | 2,0 | 2,0 | 0,3 |
| Sorbate de potassium | 0 | 0,7 | 0,1 |
| Lot 1 | **0** | **1,0** | **4,0** |
| Huile de tournesol | 40,0 | 40,0 | 30,0 |
| Amidon PGHV | 1,0 | 4,0 | 1,0 |
| Sirop de glucose | 8,0 | 8,0 | 1,5 |
| Huile d'holive | 1,5 | 3,0 | 0 |
| PREGEFLO® CH20 | 0 | 0 | 2,0 |
| Eau | 0 | 6,5 | 34,1 |
| Total : | **95** | **100** | **100** |

Croquettes pour chiens

|  | Recette initiale | Chlorelle 1% | Chlorelle 3% | Chlorelle 5% |
|---|---|---|---|---|
| Farine de volaille | 83,5 | 82,5 | 80,5 | 78,5 |
| Matière grasse de volaille | 3,5 | 3,5 | 3,5 | 3,5 |
| Saccharose | 5,0 | 5,0 | 5,0 | 5,0 |
| Sel | 1,0 | 1,0 | 1,0 | 1,0 |
| Lot 1 | 0 | 1 | 3 | 5 |
| Eau | 7 | 7 | 7 | 7 |
| Total : | 100,0 | 100,0 | 100,0 | 100,0 |
| Vit B12/repas(Beagle)(pour 240g) | 0,0 | 2,4 | 7,2 | 12,0 |
| Vit B12/repas(Labrador)(pour 500g) | 0,0 | 5,0 | 15,0 | 25,0 |

*Mélange des poudres + matière grasse*

*Extrusion avec un apport d'eau suffisant pour la cuisson des croquettes*

*T°C cuisson : 115-130°C*

*~240 g de croquettes pour des chiens de 10 à 12kg type Beagle*

*~500 g de croquettes pour des chiens de ~40kg type Labrador*

*Croquettes aux légumes*

|  | Recette initiale | Chlorelle | Chlorelle | Chlorelle | Chlorelle | Steak (100g) |
|---|---|---|---|---|---|---|
| NUTRALYS® F85G TVP | 17,0 | 17,0 | 17,0 | 17,0 | 17,0 | 17,0 |
| Eau | 74,5 | 72,2 | 72,2 | 71,2 | 69,9 | 70,8 |
| Albumen | 3,7 | 3,7 | 3,7 | 3,7 | 3,7 | 3,7 |
| Pregeflo MI20A | 2,0 | 2,0 | 2,0 | 2,0 | 2,0 | 2,0 |
| Arôme poulet | 2,5 | 0 | 0 | 0 | 0 | 0 |
| Oignons poudre | 0 | 0,60 | 0,60 | 0,60 | 0,60 | 0,60 |
| Sel | 0,3 | 0 | 0 | 0 | 0 | 0 |
| Lot 1 | **0** | **0,5** | **1** | **2** | **3,3** | **2,0** |
| Carotte poudre | 0 | 0 | 0 | 0 | 0 | 0 |
| Tomate poudre | 0 | 0 | 0 | 0 | 0 | 0 |
| Epinard poudre | 0 | **4,0** | **3,5** | **3,5** | **3,5** | **3,5** |
| Persil poudre | 0 | 0 | 0 | 0 | 0 | 0,3 |
| Muscade | 0 | 0 | 0 | 0 | 0 | 0,1 |
| Colorant | 0 | 0 | 0 | 0 | 0 | 0 |
| Total : | *100,0* | *100,0* | *100,0* | *100,0* | *100,0* | *100,0* |
| *Vit B12/repas (60ou100g)* | *0,0* | *0,3* | *0,6* | *1,2* | *2,0* | *2,0* |
| ***Apport Journalier*** | *0%* | *15%* | *30%* | *60%* | *99%* | *100%* |

*Batter :*

*Amidon de pois 43,5% ;*
*PREGEFLO® CH2020 1,2% ;*
*Sel 1,5% ;*
*Eau 53,8%*

*Préparer un bouillon de légumes : 1 cube dans 300mL d'eau bouillante*
*Hydrater les TVP 98% Nutralys (70g) dans ce bouillon pendant 30 min*
*Cutterer au blender (1min, en 2 fois) pour obtenir un aspect fibreux*
*Ajouter le mélange des poudres*
*Former les croquettes (10g) et cuire au four vapeur pendant 30 min*
*Surgeler*
*Plonger les croquettes dans le batter, puis dans la chapelure*
*Frire à 190 °C pendant 1 min*
*Surgeler*
*Cuire les nuggets à la friture à 190 °C pendant 3 min 30*

On considère que le repas est composé de 6 "croquettes" de 10 g. L'apport en VitB12 recommandé chaque jour est de 2 g (au min)

Le tableau 11 suivant présente les résultats de ces conditions d'incorporation des granules de farine de microalgues conformes à l'invention comme compléments alimentaires, en regard de leurs propriétés fonctionnelles.

Tableau 11

| Applications | | Dose de granules de farine de biomasse de microalgues (Lot 1) | | | | Avantages |
|---|---|---|---|---|---|---|
| | Cible | En g | | En % | | |
| | | Par portion | Pour 100g | Par portion | Pour 100g | |
| Soupe aux poireaux | nutrition | 1,5 | 0,5 | 0,5 | 0,5 | dispersion plus facile, meilleure répartition |
| Soupe aux légumes (Glutamate) | nutrition | 0,2 | 0,1 | 0,06 | 0,1 | dispersion plus facile, meilleure répartition |
| Cake | nutrition décor | 0,97 | 3,1 | 3,1 | 3,1 | broyage pour obtenir des granulés 100% chlorelle pour cake moucheté |
| Biscuits | nutrition | | 0,9 | | 0,9 | broyage grossier pour obtenir des granulés 100 % biomasse de microalgues pour cake moucheté |
| Ketchup | colorant | | 0,5 | | 0,5 | neutre |
| Mayonnaise | colorant | | 0,5 | | 0,5 | neutre |
| « Wine gums » | nutrition colorant | | 1 | | 1 | dispersion plus facile du produit dans une masse visqueuse |
| Cacahuètes enrobées | colorant | | 3 | | 3 | mélange des poudres et répartition facilitée |
| Sauce pesto | colorant | | 1 | | 1 | neutre |
| Croquette de légumes (2%) | colorant | 1,2 | 2 | 1,2 | 2 | neutre |
| Croquettes pour chiens | nutrition | | | | 15 | facilite l'alimentation de l'extrudeur |

De bons résultats ont été obtenus en incorporant les granules de farine de biomasse de microalgues selon l'invention dans des recettes de :

- céréales pour petit déjeuner

- yaourts

- paillettes pour assaisonnement

- crème dessert (pistache)

- croquettes pour alevin

- croquettes pour chevaux.

**Exemple 8. Formulation des granules de farine de biomasse de microalgues en compléments alimentaires**

10 recettes similaires à celles de l'exemple 7 ont été élaborées à partir des granules du Lot 3.

*Biscuits (recette classique et aux céréales)*

| | Témoin recette classique | Lot 3 recette classique | Contrôle recette Céréales | Lot 3 recette Céréales |
|---|---|---|---|---|
| Eau | 60 | 60 | 65 | 65 |
| Bicarbonate de soude | 3 | 3 | 3,5 | 3,5 |
| Bicarbonate d'ammonium | 2 | 2 | 1,5 | 1,5 |
| Saccharose | 180 | 180 | 150 | 150 |
| Miel | 0 | 0 | 25 | 25 |
| Sirop de glucose 4779 | 25 | 25 | 0 | 0 |
| MGr, Biscuitine 500 | 130 | 130 | 150 | 150 |
| Lécithine de soja | 2 | 2 | 2 | 2 |
| Farine de blé | 576 | **466** | 435 | **330** |
| Farine de blé complet | 0 | 0 | 65 | 64 |
| Flocons d'avoine | 0 | 0 | 80 | 80 |
| Lot 3 | 0 | **110** | 0 | **105** |
| Poudre de lait écrémé | 15 | 15 | 15 | 15 |
| Sel | 2 | 2 | 2 | 2 |
| Pyrophosphate de sodium | 2 | 2 | 3 | 3 |
| Arôme vanille | 2 | 2 | 2 | 2 |
| Arôme beurre *M_ 0056299* | 1 | 1 | 1 | 2 |
| | **1000** | **1000** | **1000** | **1000** |

*Pains*

|  | Témoin | NUTRALYS® Pea-BF | Lot 3 + NUTRALYS® Pea-BF | Lot 3 |
|---|---|---|---|---|
| Farine de blé | 970 | 860 | 860 | 860 |
| Gluten | 30 | 40 | 40 | 40 |
| Lot 3 | 0 | 0 | 40 | 100 |
| NUTRALYS® pea based (BF) | 0 | 100 | 60 | 0 |
| Sel | 18 | 18 | 18 | 18 |
| Levures sèches | 7 | 7 | 7 | 7 |
| Acide ascorbique | 0,2 | 0,2 | 0,2 | 0,2 |
| NUTRILIFE® AM17, enzyme | 0,2 | 0,2 | 0,2 | 0,2 |
| Eau (20°C) | 600 | 725 | 725 | 700 |
|  | **1625,4** | **1750,4** | **1750,4** | **1725,4** |

*Céréales petit déjeuner*

| % | **Témoin** | **Nutralys pea protein + Lot 3** |
|---|---|---|
| Farine de blé complet | 47,6 | 39,9 |
| Semoule de maïs | 10,5 | 5,6 |
| Amidon de maïs | 8,4 | 4,2 |
| NUTRALYS® W wheat protein | 0,0 | 12,0 |
| Lot 3 | - | 5,5 |
| Saccharose | 15,5 | 15,5 |
| Sirop de glucose 7080 | 15,0 | 15,0 |
| Eau | 3,0 | 3,0 |
|  | 100,0 | 100,0 |

*Velouté potiron*

|  | Témoin | NUTRALYS® S85F | Lot 3 | NUTRALYS® S85F + Lot 3 | NUTRALYS® S85F + Lot 3 |
|---|---|---|---|---|---|
| En % | % | % | % | % | % |
| Eau | 53,25 | 52,15 | 51,75 | 52,00 | 50,25 |
| Pommes de terre | 2 | 2 | 2 | 2,00 | 2,00 |
| Carottes | 8 | 8 | 8 | 8,00 | 8,00 |
| Potiron | 23,5 | 23,5 | 23,5 | 23,50 | 23,50 |
| Oignons émincé | 3 | 3 | 3 | 3,00 | 3,00 |
| Poireaux | 3 | 3 | 3 | 3,00 | 3,00 |

(suite)

| En % | Témoin | NUTRALYS® S85F | Lot 3 | NUTRALYS® S85F + Lot 3 | NUTRALYS® S85F + Lot 3 |
|---|---|---|---|---|---|
| En % | % | % | % | % | % |
| Crème (35% matière grasse) | 2,6 | 2,6 | 2,6 | 2,60 | 2,60 |
| CLEARAM® CH2020 | 0,8 | 0,8 | 0,8 | 0,80 | 0,80 |
| Sucre | 1 | 1 | 1 | 1,00 | 1,00 |
| Beurre (82% matière grasse) | 1,1 | 1,1 | 1,1 | 1,10 | 1,10 |
| Sel | 0,7 | 0,7 | 0,7 | 0,70 | 0,70 |
| Fibres de pois I50M | 0,8 | 0,8 | 0,8 | 0,80 | 0,80 |
| Extrait de levures | 0,2 | 0,2 | 0,2 | 0,20 | 0,20 |
| Lot 3 | - | - | 1,5 | - | - |
| NUTRALYS® S85F pea protein | - | 1,1 | - | - | - |
| Mélange 60 % NUTRALYS S85F pea protein / 40 % Lot 3 | 0 | - | - | 1,25 | 3,00 |
| Noix de muscade | 0,05 | 0,05 | 0,05 | 0,05 | 0,05 |
|  | 100 | 100 | 100 | 100 | 100,00 |

Velouté poireaux /pommes de terre

| En % | Témoin | NUTRALYS® S85F + Lot 3 | NUTRALYS® S85F + Lot 3 |
|---|---|---|---|
| En % | % | % | % |
| Eau | 50,6 | **49,9** | 48,25 |
| Pommes de terre | 14,5 | 14,5 | 14,5 |
| Epinards | 5,5 | 5,5 | 5,5 |
| Oignons émincé | 4,4 | 4,4 | 4,4 |
| Poireaux | 18,0 | 18,0 | 18 |
| Crème (35% matière grasse) | 2,2 | 2,2 | 2,2 |
| CLEARAM® CH2020 | 0,8 | 0,8 | 0,6 |
| Sucre | 1,0 | 1,0 | 1 |
| Beurre (82% matière grasse) | 1,5 | 1,5 | 1,5 |
| Sel | 0,7 | 0,7 | 0,8 |
| Fibres de pois I50M | 0,8 | 0,8 | 0,8 |
| Mélange 60 % NUTRALYS S85F pea protein / 40 % Lot 3 | 0,0 | **0,7** | 2,45 |
|  | 100 | 100 | 100 |

Mix pour boissons à 21 % protéines

|  | Témoin | Lot 3 | NUTRALYS® S85F + Lot 3 |
|---|---|---|---|
| En % | % | % | % |
| Poudre de lait écrémé | 28,5 | 30,2 | 28,6 |
| Sucre | 27,0 | 27,0 | 27,0 |
| GLUCIDEX® IT 47 | 23,5 | 20,3 | 22,4 |
| Poudre de cacao (23-24% matière grasse) | 6,0 | 6,0 | 6,0 |
| NUTRALYS® XF | 4,0 | 0,0 | 5,4 |
| NUTRALYS® WF | 4,0 | 0,0 | 0,0 |
| Lot 3 | 0,0 | 9,5 | 3,6 |
| WPC 90 | 4,0 | 4,0 | 4,0 |
| Pregeflo® C100 | 3,0 | 3,0 | 3,0 |
| Total | 100,0 | 100,0 | 100,0 |

*Mix pour crème chocolat*

|  | Témoin | Rapport NUTRALYS® XF (60%) Lot 3 (40%) | |
|---|---|---|---|
|  |  | B -Isolat protéines de lait substitution | C - Poudre de lait entier substitution |
| En % | % | % | % |
| Isolat protéines de lait (Prodiet 85_Ingredia) | 36,5 | 0,00 | 36,5 |
| NUTRALYS® XF_Lab 4460 (W106R) | 0,00 | 24,50 | 6,00 |
| HPAF (EA342) | 0,00 | 16,30 | 4,00 |
| Poudre de lait entier | 27,00 | 27,00 | 0,00 |
| Poudre de cacao « low fat » | 13,00 | 13,00 | 13,00 |
| GLUCIDEX® IT19 | 0,00 | 0,00 | 17,00 |
| PREGEFLO® C100 | 5,00 | 3,00 | 5,00 |
| NUTRIOSE® FM06 | 6,00 | 3,70 | 6,00 |
| Caramel colorant (M_0052208) | 2,00 | 2,00 | 2,00 |
| carraghénanes ( Matgum L03_AGI) | 3,70 | 3,70 | 3,70 |
| Gomme de guar (Matguar 5000_AGI) | 1,11 | 1,11 | 1,11 |
| Gomme de xanthanes (F80_AGI) | 1,11 | 1,11 | 1,11 |
| Sel | 1,50 | 1,50 | 1,50 |
| Aspartame | 0,40 | 0,40 | 0,40 |
| Potassium acesulfame | 0,20 | 0,20 | 0,20 |
| Mix de Vitamines PNU CN 02_VitaBlend | 0,12 | 0,12 | 0,12 |
| Mix de Minéraux PNU CN 02_VitaBlend | 2,36 | 2,36 | 2,36 |
| Total | 100,00 | 100,00 | 100,00 |

*Boisson 100% végétale à base*

| | Lot 3 |
|---|---|
| En % | **%** |
| NUTRALYS® S85F pea protein | 2,81 |
| Biomasse de Chlorelle riche en lipides | 1,70 |
| Lot 3 | 1,87 |
| Saveur de masquage SYMRISE 688571 | 0,187 |
| Saveur poire SYMRISE 329082 | 0,204 |
| Arôme vanille SYMRISE 826892 | 0,041 |
| CLEARGUM® CK2020 | 0,26 |
| Sucre de cane | 3,40 |
| Eau | 89,53 |
| | 100 |

Le tableau 12 suivant présente les résultats de ces conditions d'incorporation des granules de farine de microalgues conformes à l'invention comme compléments alimentaires, en regard de leurs propriétés fonctionnelles.

Tableau 12

| Application | Cibles | Allégation | % Protéines totales | % Farine d'aigues riche en proteines | Autres protéines formule | Avantages |
|---|---|---|---|---|---|---|
| PRODUITS PANIFICATION | Biscuit classique | source | 14,1 | 11 | blé | Pas de modification is recette, le procédé au le temps de cuisson texture du biscuit non modifié, gout correct |
| | Biscuit céréales | source | 14,3 | 10,5 | blé | Pas de modification la recette, le procédé ou le temps de cuisson. texture du biscuit non modifié, gout correct |
| | Pain | riche | | 5.7 | blé | Pas de modification de recette, pas de modification process. pates similaires au témoin. |
| | | | | 2,3 | blé, pois | Pas d'influence majeurs sur la texture des pains, coloration légérement plus forte. |
| | Céréales petit déjeuner | riche | 19,3 | 5,5 | pois | ok en gout et croustillance |

(suite)

| Application | Cibles | Allégation | % Protéines totales | % Farine d'aigues riche en proteines | Autres protéines formule | Avantages |
|---|---|---|---|---|---|---|
| SOUPES | velouté potiron | source | 1,4 | 0,5 | pois | Les formules de soupes enrichies en protéines donnent de meilleurs résultats en utilisant un mix 80% de Nutralys®S85F / 40% lot 3, tant par rapport à la texture qu'au goût Ce ratio 60/40 est d'autant plus intéressant qu'il permet d'optimiser le profil en acides aminés (notion de PDCAAS). Le lot 3 et le NUTRALYS® S85F résistent au traitement thermique |
| | | riche | 2,6 | 1,2 | pois | |
| | Velouté poireaux-pomme de terre | source | 1,5 | 0,3 | pois | |
| | | riche | 2,8 | 1 | | |
| NUTRITION | Mix pour boisson à 21% protéines | | 21,5 | 3,6 | lactosérum, pois lait écrémè | Bonne consistance et corps; gout ok |
| | Mix pour crème chocolat | | 39,9 | 4 | lait isolat, pois | Bonne texture et gout acceptable |
| | Boisson 100% vègétale à base de farine d'algues | | 3,51 | 1,87 | pois | Mélange farine riches en lipdes et protéines et de NUTRALYS® |

**Exemple 9. Incorporation des granules de farine de biomasse de microalgues dans des barres hyperprotéinées.**

Dans cet exemple, on réalise des barres hyperprotéinées combinant les granules de farine de biomasse de *Chlorella protothecoides* (Lot 3 de l'exemple 5) avec d'autres ingrédients commercialisés par la société Demanderesse selon les recettes présentées dans le tableau ci-dessous.

*Barres hyperprotéinées*

| | Témoin | Lot 3 | 60/40 NUTRALYS® XF+BF / Lot 3 |
|---|---|---|---|
| En % | % | % | % |
| FLOLYS® E7081S | 21,10 | 21,10 | 20,25 |
| Matière grasse | 3,00 | 3,00 | 2,90 |
| Lot 3 | - | 37,44 | 16,10 |
| Poudre de cacao | 0,90 | 0,90 | 0,90 |

(suite)

| | Témoin | Lot 3 | 60/40 NUTRALYS® XF+BF / Lot 3 |
|---|---|---|---|
| En % | % | % | % |
| Chocolat maltitol | 15,00 | - | - |
| NEOSORB® 70/70 | 1,75 | 1,75 | 1,68 |
| NUTRALYS® XF+BF | 21,40 | - | 14,64 |
| NUTRALYS® WF | 15,20 | - | 9,98 |
| chocolat sucre | 0,00 | 15,00 | 15,00 |
| NUTRIOSE® 06 | 7,20 | 7,20 | 7,24 |
| Eau | 12,80 | 12,80 | 10,17 |
| Total | 98,35 | 99,19 | 98,86 |

**Conclusions :**

Il est observé la formation d'une pâte souple très rapidement pendant le pétrissage ; et lors de l'incorporation de protéines de blé et de pois, la texture est qualifiée de souple, masticable et non granuleuse. Le goût « céréales grillées » est jugé agréable.

**Exemple 10. Incorporation des granules de farine de biomasse de microalgues dans des boissons pour sportifs.**

Dans cet exemple, on réalise des boissons hyperprotéinées pour sportifs combinant les granules de farine de biomasse de *Chlorella protothecoides* (Lot 3 de l'exemple 5) avec d'autres ingrédients commercialisés par la société Demanderesse selon les recettes présentées dans le tableau ci-dessous.

*Mix pour boissons à 74% protéines*

| | **Témoin** | Lot 3 | NUTRALYS® S85F + Lot 3 |
|---|---|---|---|
| En % | % | % | % |
| Isolat protéique de lactosérum WPC 80 | 49,62 | 69,46 | 49,62 |
| **NUTRALYS® XF** | 49,62 | - | **29,77** |
| Lot 3 | - | **29,78** | **19,85** |
| REBAUDIOSIDE A (Stevia) | 0,06 | 0,06 | 0,06 |
| Arôme vanille MANE M555943 | 0,70 | 0,7 | 0,7 |
| | **100** | **100** | **100** |

**Conclusions :**

Les formules sont bien adaptées : si le Gout reste végétal, il n'y a pas d'amertume. La farine de biomasse de *Chlorella protothecoides* confère même une Rondeur en bouche agréable.

**Revendications**

1. Granules de farine de biomasse de microalgues **caractérisés en ce qu'**ils sont obtenus à partir d'une biomasse de microalgues du genre *Chlorella* comprenant au moins 50% en poids sec de protéines, de préférence entre 50 et 70% en poids sec de protéines, et **en ce qu'**ils présentent :

◦ une distribution granulométrique, mesurée sur un granulomètre laser LS de marque COULTER®, présentant un Dmode compris entre 60 et 300 μm et un D4,3 entre 70 et 420 μm,
◦ une densité aérée, mesurée sur Powder Characteristics Tester HOSOKAWA, comprise entre 0,60 et 0,70 g/ml,
◦ une compressibilité, mesurée sur Powder Characteristics Tester HOSOKAWA, comprise entre 15 et 25 %, de préférence entre 18 et 21 %.

2. Granules selon la revendication 1, **caractérisés en ce que** la microalgue est choisie dans le groupe constitué de *Chlorella sorokiniana* et *Chlorella protothecoides.*

3. Granules selon la revendication 2, **caractérisés en ce que** la microalgue est *Chlorella sorokiniana.*

4. Granules selon la revendication 2, **caractérisés en ce que** la microalgue est *Chlorella protothecoides.*

5. Granules selon la revendication 3, **caractérisés en ce qu'**ils présentent une distribution granulométrique présentant un Dmode compris entre 70 et 130 μm et un D4,3 entre 75 et 140 μm, de préférence un Dmode compris entre 200 et 280 μm et un D4,3 entre 300 et 420 μm.

6. Granules selon la revendication 1, **caractérisés en ce qu'**ils présentent un degré de mouillabilité, exprimé selon un test B, par la hauteur du produit décanté dans un bécher, à une valeur comprise entre 5 et 25 mm, de préférence entre 5 et 15 mm ou entre 15 et 25 mm.

7. Granules selon l'une quelconque des revendications 1 à 6, **caractérisés en ce qu'**ils présentent une surface spécifique selon la méthode BET, comprise entre 0,45 et 0,70 m$^2$/g, de préférence comprise entre 0,45 et 0,50 m$^2$/g ou entre 0,60 et 0,70 m$^2$/g.

8. Procédé de préparation des granules selon l'une quelconque des revendications 1 à 7, **caractérisé en ce qu'**il comprend les étapes suivantes :

1) préparer une suspension de biomasse de microalgues du genre *Chlorella* comprenant au moins 50% en poids sec de protéines, de préférence entre 50 et 70% en poids sec de protéines, dans de l'eau à une matière sèche comprise entre 10 et 35 % en poids sec,
2) la pulvériser dans un atomiseur vertical équipé d'une bande mobile à sa base, et d'une buse haute pression à sa partie supérieure, tout en réglant :

• la première température de l'air primaire à une valeur comprise entre 160 et 220°C,
• la seconde température de l'air primaire à une valeur comprise entre 90 et 150°C
• la pression de pulvérisation à une valeur comprise entre 50 et 250 bars, de préférence entre 80 et 150 bars,

3) régler la température d'entrée de la zone de post séchage sur la bande mobile à une valeur comprise entre 70 et 90°C, et régler la température de la zone de refroidissement à une valeur comprise entre 15 et 25°C,
4) collecter les granules de farine de biomasse de microalgues ainsi obtenus.

9. Utilisation des granules de l'une quelconque des revendications 1 à 7 ou obtenus selon le procédé de la revendication 8 en alimentations humaine et animale ou pour des applications dans l'industrie pharmaceutique et cosmétique.

10. Utilisation selon la revendication 9, pour la fabrication de comprimés orodispersibles.

11. Utilisation selon la revendication 9, pour la fabrication de soupes, sauces, gâteaux, biscuits, céréales pour petit déjeuner, mayonnaise, ketchup, « wine gums », cacahuètes enrobées, yaourts, crème dessert, nuggets de légumes, paillettes pour assaisonnement.

12. Utilisation selon la revendication 9, pour la fabrication de barres hyperprotéinées.

13. Utilisation selon la revendication 9, pour la fabrication de boissons hyperprotéinées pour sportifs.

14. Utilisation selon la revendication 9, pour la fabrication de croquettes pour chiens, croquettes pour chevaux, ou croquettes pour alevins.

**Patentansprüche**

1.  Granulat aus Mikroalgenbiomassemehl, **dadurch gekennzeichnet, dass** es aus einer Biomasse von Mikroalgen der Gattung *Chlorella* erhalten ist, umfassend wenigstens 50 Trockengew.-% Proteine, vorzugsweise zwischen 50 und 70 Trockengew.-% Proteine, und dass es aufweist:

    ◦ eine granulometrische Verteilung, gemessen auf einem LS Laser-Granulometer der Marke COULTER®, die einen Dmode zwischen 60 und 300 $\mu$m und einen D4,3 zwischen 70 und 420 $\mu$m aufweist,
    ◦ eine belüftete Dichte, gemessen auf einem HOSOKAWA Powder Characteristics Tester, zwischen 0,60 und 0,70 g/ml,
    ◦ eine Kompressibilität, gemessen auf einem HOSOKAWA Powder Characteristics Tester, zwischen 15 und 25%, vorzugsweise zwischen 18 und 21%.

2.  Granulat gemäß Anspruch 1, **dadurch gekennzeichnet, dass** die Mikroalge aus der Gruppe ausgewählt ist, bestehend aus *Chlorella sorokiniana* und *Chlorella protothecoides.*

3.  Granulat gemäß Anspruch 2, **dadurch gekennzeichnet, dass** die Mikroalge *Chlorella sorokiniana* ist.

4.  Granulat gemäß Anspruch 2, **dadurch gekennzeichnet, dass** die Mikroalge *Chlorella protothecoides* ist.

5.  Granulat gemäß Anspruch 3, **dadurch gekennzeichnet, dass** es eine granulometrische Verteilung aufweist, die einen Dmode zwischen 70 und 130 $\mu$m und einen D4,3 zwischen 75 und 140 $\mu$m, vorzugsweise einen Dmode zwischen 200 und 280 $\mu$m und einen D4,3 zwischen 300 und 420 $\mu$m aufweist.

6.  Granulat gemäß Anspruch 1, **dadurch gekennzeichnet, dass** es einen Grad an Benetzbarkeit, ausgedrückt nach einem Test B durch die Höhe des abgesetzten Produkts in einem Becherglas mit einem Wert zwischen 5 und 25 mm, vorzugsweise zwischen 5 und 15 mm oder zwischen 15 und 25 mm, aufweist.

7.  Granulat gemäß einem der Ansprüche 1 bis 6, **dadurch gekennzeichnet, dass** es eine spezifische Oberfläche nach dem BET-Verfahren zwischen 0,45 und 0,70 m$^2$/g, vorzugsweise zwischen 0,45 und 0,50 m$^2$/g oder zwischen 0,60 und 0,70 m$^2$/g, aufweist.

8.  Verfahren zur Herstellung des Granulats gemäß einem der Ansprüche 1 und 7, **dadurch gekennzeichnet, dass** es die folgenden Schritte umfasst:

    1) Herstellen einer Biomasse-Suspension von Mikroalgen der Gattung *Chlorella,* umfassend wenigstens 50 Trockengew.-% Proteine, vorzugsweise zwischen 50 und 70 Trockengew.-% Proteine, in Wasser mit einer Trockenmasse zwischen 10 und 35 Trockengew.-%,
    2) Sprühen der Suspension in einen vertikalen Sprühtrockner, der mit einem beweglichen Band an seiner Basis und einer Hochdruckdüse in seinem oberen Teil ausgestattet ist, unter Regulierung:

    • der ersten Temperatur der Primärluft auf einen Wert zwischen 160 und 220°C,
    • der zweiten Temperatur der Primärluft auf einen Wert zwischen 90 und 150°C,
    • des Sprühdrucks auf einen Wert zwischen 50 und 250 bar, vorzugsweise zwischen 80 und 150 bar,

    3) Regeln der Eingangstemperatur der Nachtrocknungszone auf dem beweglichen Band auf einen Wert zwischen 70 und 90°C und Regeln der Temperatur der Kühlungszone auf einen Wert zwischen 15 und 25°C,
    4) Sammeln des auf diese Weise erhaltenen Granulats aus Mikroalgenbiomassemehl.

9.  Verwendung des Granulats nach einem der Ansprüche 1 bis 7 oder des nach dem Verfahren von Anspruch 8 erhaltenen Granulats in Tier- und Humannahrungsmitteln oder für Anwendungen in der kosmetischen und pharmazeutischen Industrie.

10. Verwendung gemäß Anspruch 9 zur Herstellung von Schmelztabletten.

11. Verwendung gemäß Anspruch 9 zur Herstellung von Suppen, Saucen, Kuchen, Keksen, Frühstückszerealien, Mayonnaise, Ketchup, "Weingummis", ummantelten Erdnüssen, Joghurt, Cremedessert, Gemüsenuggets, Gewürzflocken.

**12.** Verwendung gemäß Anspruch 9 zur Herstellung von proteinreichen Riegeln.

**13.** Verwendung gemäß Anspruch 9 zur Herstellung von proteinreichen Getränken für Sportler.

**14.** Verwendung gemäß Anspruch 9 zur Herstellung von Kroketten für Hunde, Kroketten für Pferde oder Kroketten für Jungfische.

**Claims**

**1.** Granules of microalgal biomass flour, **characterized in that** they are obtained from a biomass of microalgae of the genus *Chlorella* comprising at least 50% by dry weight of proteins, preferably between 50 and 70% by dry weight of proteins, and **in that** they have:

  ◦ a particle size distribution, measured on an LS laser particle size analyzer of the COULTER® brand, having a Dmode of between 60 and 300 $\mu$m and a D4,3 between 70 and 420 $\mu$m,
  ◦ a bulk density, measured on a HOSOKAWA Powder Characteristics Tester, of between 0.60% and 0.70 g/ml,
  ◦ a compressibility, measured on a HOSOKAWA Powder Characteristics Tester, of between 15% and 25%, preferably between 18% and 21%.

**2.** The granules as claimed in claim 1, **characterized in that** the microalga is chosen from the group consisting of *Chlorella sorokiniana* and *Chlorella protothecoides.*

**3.** The granules as claimed in claim 2, **characterized in that** the microalga is *Chlorella sorokiniana.*

**4.** The granules as claimed in claim 2, **characterized in that** the microalga is *Chlorella protothecoides.*

**5.** The granules as claimed in claim 3, **characterized in that** they have a particle size distribution having a Dmode of between 70 and 130 $\mu$m and a D4,3 between 75 and 140 $\mu$m, preferably a Dmode of between 200 and 280 $\mu$m and a D4,3 between 300 and 420 $\mu$m.

**6.** The granules as claimed in claim 1, **characterized in that** they have a degree of wettability, expressed according to a test B, by the height of the product decanted in a beaker, at a value of between 5 and 25 mm, preferably between 5 and 15 mm or between 15 and 25 mm.

**7.** The granules as claimed in any one of claims 1 to 6, **characterized in that** they have a specific surface area, according to the BET method, of between 0.45 and 0.70 m$^2$/g, preferably between 0.45 and 0.50 m$^2$/g or between 0.60 and 0.70 m$^2$/g.

**8.** A process for preparing the granules as claimed in any one of claims 1 to 7, **characterized in that** it comprises the following steps:

  1) preparing a suspension of biomass of microalgae of the genus *Chlorella* comprising at least 50% by dry weight of proteins, preferably between 50 and 70% by dry weight of proteins, in water at a solids content of between 10% and 35% by dry weight,
  2) spraying it in a vertical spray-drier equipped with a moving belt at its base, and with a high-pressure nozzle in its upper part, while regulating:

    • the first temperature of the primary air at a value of between 160 and 220°C,
    • the second temperature of the primary air at a value of between 90 and 150°C,
    • the spray pressure at a value of between 50 and 250 bar, preferably between 80 and 150 bar,

  3) regulating the entry temperature of the post-drying zone on the moving belt at a value of between 70 and 90°C, and regulating the temperature of the cooling zone at a value of between 15 and 25°C,
  4) collecting the granules of microalgal biomass flour thus obtained.

**9.** The use of the granules of any one of claims 1 to 7 or obtained according to the process of claim 8 in food for human consumption and animal feed or for applications in the pharmaceutical and cosmetics industry.

10. The use as claimed in claim 9, for producing orodispersible tablets.

11. The use as claimed in claim 9, for producing soups, sauces, cakes, cookies, breakfast cereals, mayonnaise, ketchup, wine gums, coated peanuts, yogurts, cream desserts, vegetable nuggets, and seasoning flakes.

12. The use as claimed in claim 9, for producing high-protein bars.

13. The use as claimed in claim 9, for producing high-protein sports drinks.

14. The use as claimed in claim 9, for producing dry dog food, dry food for horses, or dry food for alevins.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6607900 B
- US 6372460 B

- DE 102006056454
- US 6255505 B

**Littérature non-brevet citée dans la description**

- **HAUGAARD SORENSEN et al.** *Méthodes d'analyse des produits laitiers déshydratés,* 1978
- **CAYOT ; LORIENT.** Structures et technofonctions des protéines du lait. *Airlait Recherches: Tec et Doc,* 1998

- **S. BRUNAUER et al.** *Journal of American Chemical Society,* 1938, vol. 60, 309